# EUROPEAN PATENT SPECIFICATION

(11) **EP 1 986 717 B1**
(45) Date of publication and mention of the grant of the patent: **09.06.2010**
(21) Application number: 07717945.5
(22) Date of filing: 06.01.2007
(51) Int. Cl.: A61M 1/00

(54) **APPARATUS FOR FACILITATING THE REPLACEMENT OF AN IMPLANTED CATHETER**
GERÄT ZUR ERLEICHTERUNG DES AUSTAUSCHENS EINES IMPLANTIERTEN KATHETERS
APPAREIL POUR FACILITER LE REMPLACEMENT D'UN CATHÉTER IMPLANTÉ

(30) Priority: 11.01.2006 US 758137 P
(43) Date of publication of application: 05.11.2008
(73) Proprietor: Medical Research Products-B Inc., Santa Clarita CA 91355 (US)
(72) Inventor: PORTER, Christopher H., Woodinville, Washington 98072 (US); VIDAL, Claude A., Santa Barbara, CA 93111 (US); REDMOND, Russ J., Goleta, CA 93117 (US); MORAN, Byron L., Santa Barbara, CA 93111 (US); KALUZNIAK, Paul, St. Paul, MN 55116 (US); JANIS, Abram D., Valencia, CA 91354 (US)
(74) Representative: Webster, Jeremy Mark
(86) International application number: PCT/US2007/000191
(87) International publication number: WO 2007/081749

(56) References cited:
- US-A- 4 344 435
- US-A- 4 781 693
- US-A- 4 781 693
- US-A- 6 156 016

## Description

### FIELD OF THE INVENTION

This invention relates generally to medical technology and more particularly to an apparatus for implanting an elongate conduit, e.g., a catheter or cable, so as to extend through a patient's skin for providing long tenn access to an interior body site. Embodiments of the invention are useful in a variety of applications, e.g., in hemodialysis procedure to provide access to the patient's central venous system.

### BACKGROUND OF THE INVENTION

In a variety of medical procedures, catheters are implanted through a patient's skin to provide long term access to interior body sites: e.g., blood vessels and organs. Unless adequate precautions are taken, infection and inflammation are likely to occur around the site where the catheter penetrates the skin. To mitigate such problems, a tissue integrating cuff is sometimes attached to the catheter and placed under the patient's skin to resist infection. Although such a cuff can reduce the likelihood of infection, its presence increases the difficulty of removing and/or repositioning the implanted catheter. More particularly, it is not uncommon for an implanted catheter to become damaged, e.g., dogged or kinked, over an extended period of use thus necessitating catheter removal and/or replacement. When this occurs, the cuff must be dissected thereby complicating and prolonging the surgical procedure. US 4781693 describes an implant device for the replacement of a flexible catheter without invasive surgery, where the catheter preferably terminates within the interior cavity of the percutaneous body.

The aforementioned Application 10/821,383 describes the use of a tissue integrating structure on a percutaneously implanted medical device for anchoring the device and creating an infection resistant barrier around the device.

### SUMMARY OF THE INVENTION

The present invention provides apparatus for percutaneous implantation in a patient's body according to claim 1. The present invention is directed to a medical apparatus for percutaneously implanting an elongate conduit, e.g., a catheter or cable, in a patient's body in a manner which allows the conduit to be easily positioned, repositioned, and replaced.

An apparatus in accordance with the invention includes an elongate sleeve comprising a wall surrounding an interior elongate passageway. The passageway extends from a sleeve proximal end to a sleeve distal end. The sleeve is intended to be percutaneously implanted through an incision in the patient's skin so that the sleeve distal end resides beneath the skin, i.e., subcutaneously, and the sleeve proximal end resides above the skin. The steeve outer peripheral surface carries a layer of porous material, e.g., a biocompatible mesh, as described in US Application 10/821,383, intended to be placed just under the patient's outer skin layer in contact with the dermis to promote tissue ingrowth for anchoring the sleeve and forming an infection resistant barrier. The sleeve passageway is dimensioned to snugly accommodate the outer surface of a conduit (which will hereinafter be assumed to be a catheter unless otherwise stated) while permitting the catheter to slide in the passageway relative to the sleeve, A sealing device extends around the catheter near the sleeve proximal end to prevent deleterious material from migrating into the patent's body along the catheter outer surface.

In accordance with one preferred embodiment, the sealing device includes a compressible annular seal which cooperates with the sleeve proximal end to close a potential migration path along the catheter outer surface. Preferably, the sleeve wall inner surface at its proximal end tapers outwardly to form a wedge recess for accommodating at least a portion of the annular seal, A locking, or compression, member is mounted around the catheter for longitudinal movement along the catheter to compress the seal in the recess between the sleeve wall and catheter outer surface. Latching means are provided for latching the compression member to the sleeve to maintain adequate compression between the seal, the sleeve wall, and the catheter outer surface.

In typical use, of an embodiment, a physician will make an incision proximate to the patient's chest or abdomen. A surgical tunneler tool is then typically inserted through the incision to form a subcutaneous tunnel to an interior site through which a catheter can be inserted. In accordance with a preferred embodiment, a sleeve, an annular seal, and a compression member, are mounted on the catheter as previously described. The distal end of the sleeve is then inserted through the incision to locate the sleeve porous layer in contact with the dermis just below the patients outer skin surface. With the sleeve anchored and the compression member in its unlocked state, the physician is able to slide and/or rotate the catheter within the sleeve for optimum catheter positioning. When the catheter is properly positioned, the annular seal is moved along the catheter against the sleeve proximal end to engage a portion In the wedge recess. The compression member can then be brought up against and latched to the sleeve thus compressing the seal therebetween and preventing relative movement between the catheter and sleeve. With the sleeve thus implanted, the patient's subcutaneous tissue will, over time, grow into the porous material to anchor the sleeve and form an infection resistant barrier. The porous material may be coated or impregnated with constituents having antimicrobial and/or anti-inflammatory properties to promote healing, e.g., silver containing compounds or antibiotic eluting coatings and/or steroids.

In one preferred embodiment of the invention, the porous layer on the sleeve is covered prior to use by a protective sheath of thin flexible material. The sheath prevents abrasion damage as the sleeve porous layer is inserted through the incision. The sheath is preferably configured with a projecting tab which allows the physician to readily peel the sheath away, e.g., along a preformed score line, as the sleeve is inserted through the incision to place the porous layer adjacent the patient's dermis.

After the sleeve and catheter have been implanted, subcutaneous tissue will gradually grow into the porous layer to form an infection resistant barrier around the sleeve to prevent fluid and/or other deleterious material from migrating into the body along the sleeve outer surface. The annular seal functions to prevent deleterious material from migrating along the catheter outer surface. An apparatus in accordance with the invention enables the physician at some later date (e.g., months) to replace the implanted catheter while leaving the sleeve in place. To do this, the physician will first unlatch the existing compression member to allow the old catheter to be withdrawn from the sleeve proximal end. A new catheter, preferably carrying a new seal and compression member, is then inserted through the sleeve. The new compression member is then latched to the existing sleeve to compress the annular seal therebetween to seal the path along the catheter outer surface.

As was previously mentioned, once the sleeve has been anchored, if the compression member is unlatched, the physician can slide and/or rotate the catheter relative to the sleeve for optimum catheter positioning, prior to latching the compression member to hold the catheter in place. In order to prevent the introduction of deleterious material into the body when the catheter is being inwardly adjusted, it is preferable to form a sterile field around that portion of the catheter which can move inwardly past the annular seal. Thus, in a preferred embodiment, an extensible seal member, e.g., a bellows like sheath, is mounted around the catheter with the distal end of the bellows sheath sealed to the compression member and the proximal end of the bellows sheath sealed to the catheter outer surface. This arrangement maintains the portion of the catheter distal from the bellows proximal end within a sterile field to avoid introducing deleterious material past the annular seal when the catheter is adjusted inwardly.

### BRIEF DESCRIPTION OF THE FIGURES

Figure 1 is a schematic representation depicting a medical device in accordance with the Invention for percutaneously implanting a catheter for an exemplary hemodialysis application;

Figure 2 is an isometric view of a preferred catheter assembly in accordance with the invention;

Figure 3 is an exploded view of the assembly of Figure 2 showing a catheter in phantom together with a protective sheath, a sleeve, a layer of porous material, a sleeve anchor, an annular seal, and a compression member;

Figure 4 is a sectional view taken substantially along the plan 4-4 of Figure 2;

Figure 5 is a plan view of the protective sheath;

Figure 6 is a sectional view taken substantially along the plan 6-6 of Figure 5 particularly showing a performed score line;

Figures 7-11 show successive steps in an exemplary procedure for implanting and utilizing the catheter assembly in accordance with the invention;

Figure 12 shows a cross-sectional view of the catheter assembly as implanted with the porous layer adjacent the patient's dermis; and

Figure 13 shows an optional extensible seal member which can be used to create a sterile field around the catheter adjacent to the sleeve proximal end to enable the catheter to be inwardly adjusted while preventing the introduction of deleterious material along the path between the catheter outer surface and the sleeve inner surface.

### DETAILED DESCRIPTION

Various medical regimens utilize a percutaneously implanted flexible elongate conduit to provide access to an interior body site. For example, hemodialysis drug infusion, plasmapheresis, and other procedures typically employ a percutaneously implanted catheter for delivering fluid to or extracting fluid from an interior body site. Other procedures utilize an electric cable to deliver an electric signal to, or extract an electric signal from, an interior body site. The present invention is directed to an apparatus for facilitating the long term implantation and utilization of a flexible elongate conduit and for facilitating the positioning, repositioning, and replacement, or exchange, of the conduit.

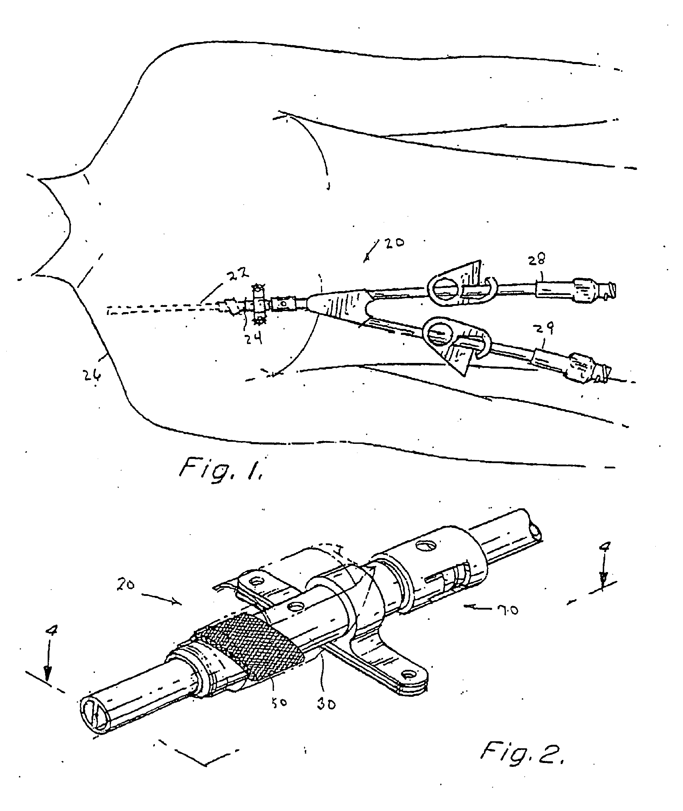

Figure 1 schematically depicts an apparatus 20 in accordance with the invention for percutaneously implanting a catheter 22 through an incision 24 in a patient 26 undergoing an exemplary hemodialysis procedure. In such a procedure, a dual lumen catheter 22 is typically used with the two lumen respectively coupled to separate exterior flow couplers 28 and 29.

Attention is now directed to Figures 2-4 which depict a preferred catheter assembly 20 in accordance with the present invention. The assembly 20 is comprised of an elongate sleeve 30 formed by a sleeve wall 32 having a peripheral outer surface 34 and a peripheral inner surface 36. The inner surface 36 surrounds a passageway 38 extending from a first, or proximal, end 40 to a second, or distal, end 42.

The sleeve 30 is shown mounted an a catheter 22 extending through the passageway 38. The catheter outer surface 44 and passageway wall surface 36 are closely dimensioned but with sufficient clearance therebetween to enable the catheter to slide axially in the passageway.

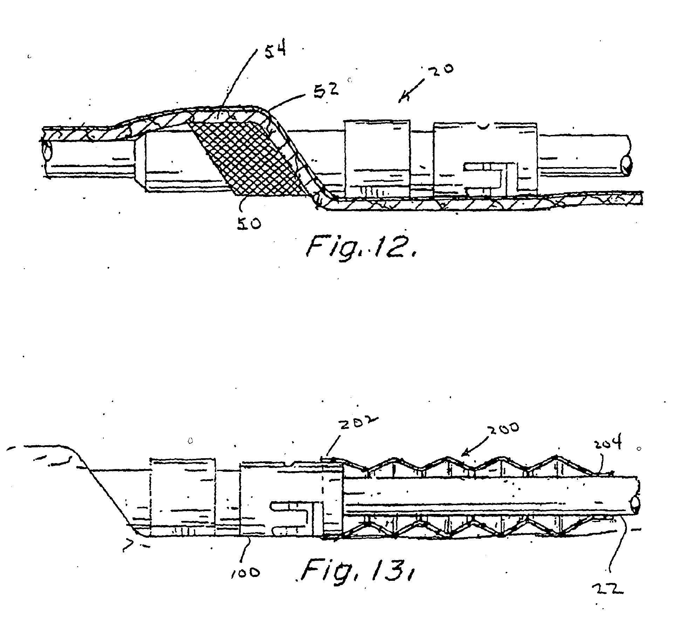

A layer 50 of porous material. e.g., titanium mesh, as described in said US Application 10/821,383, is mounted on the sleeve 30 close to the sleeve distal end 42. In use, it is intended that the sleeve distal end be inserted through an incision in the patient's skin to position the porous layer 50 just below the patient's epidermis skin layer 52 and adjacent to the patient's dermis layer 54 (Figure 12) in contact with the patient's dermis layer 54. Note that the porous layer 50 is preferably oriented diagonally with respect to the axis of sleeve 30 to better conform to the patient's skin contour (Figure 12). This orientation optimizes contact between the porous layer 50 and the patient's dermis to promote, over time soft tissue ingrowth into the porous layer. This tissue ingrowth acts to form an infection resistant barrier around sleeve 30. This barrier may be enhanced by incorporating antimicrobial and/or anti-inflammatory constituents into the porous layer 50. For example, silver containing compounds and/or antibiotic eluting coatings can be used as antimicrobial agents and steroids can be used as anti-inflammatory agents.

A protective sheath 56 is preferably mounted around sleeve 30 and porous layer 50 prior to use to avoid tissue abrasion damage when the sleeve distal end 42 is inverted through the patient's incision. The protective sheath 56 is preferably formed of thin flexible tubular material (e.g., 0.254 mm [.010] wall FEP tubing). As win be further discussed hereinafter, the sheath 56 is removed from the sleeve 30 by the physician after the sleeve and porous layer have been inserted through the incision.

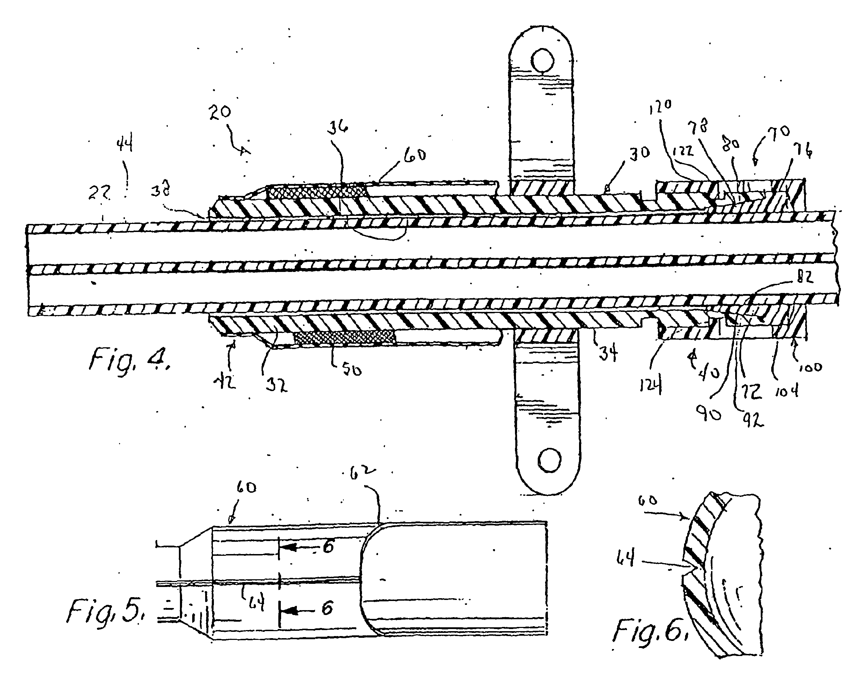

More particularly, the sheath 56 is preferably configured as a substantially tubular, e.g., cylindrical, body 58 having a distal collar 59 and a proximal elongate pull tab 60. An outwardly tapering section 61 extends from the collar 59 to the main body portion 58. Note that the collar 59 and distal portion of section 61 have a diameter smaller than that of the porous layer 50. For example only, the sleeve 30 may have an outer diameter of [6.35 mm 250 inches], the porous layer 50 an outer diameter of [7.87 mm 310 inches] and the collar 59 an inner diameter of [4.90 mm 193 inches.] An axially oriented score, or perforated line 62 is preformed through the collar 59, the tapering section 61 and the body portion 58 to facilitate the physician peeling the sheath 56 from the sleeve 30. Note in Figure 4 that the sheath fits tightly around the periphery of sleeve 30 and porous layer 50 and that the tapering section 61 is positioned distally of the porous layer 50. In use, the physician is able to readily peel the sheath from the sleeve with one hand by rolling, or winding, the elongate tab 60 to pull the sheath axially in a proximal direction. Peeling occurs because the sheath is putted proximally, the tapering section 61 and collar 59 have to move past the larger diameter porous layer 50 which action causes the sheath to tear along score line 62 allowing it to be easily stripped from the sleeve 30.

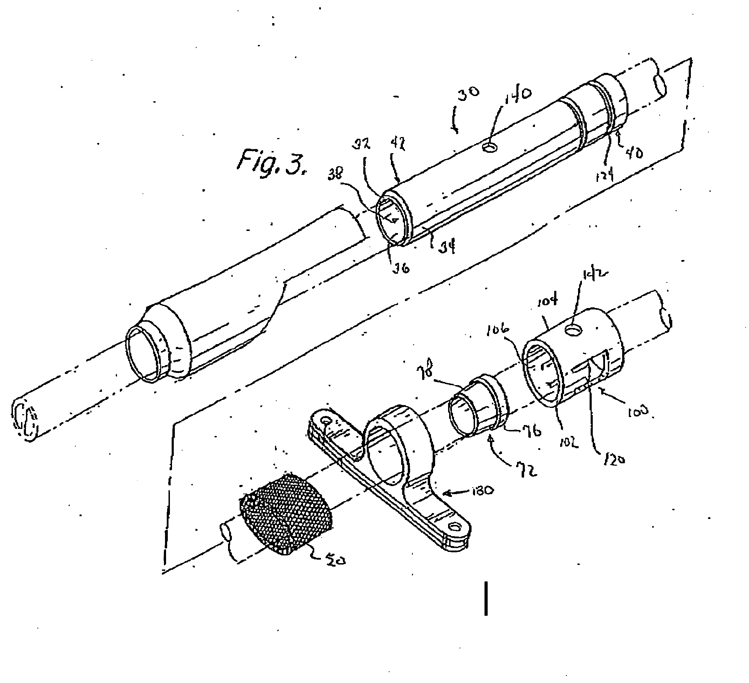

As has previously been mentioned, in use, dermis tissue grows into the porous layer 50 to form a barrier preventing deleterious material from migrating into the patient's body along the sleeve outer surface 34. In order to prevent migration of deleterious material into the body along the narrow gap between the catheter outer surface 44 and the sleeve inner surface 36, a sealing device 70 is provided. A preferred sealing device 70 is comprised of a seal member 72, preferably in the form of a compressible annular member or ring. The seal member 72, as shown in Figures 3 and 4, includes an end flange 76 having a distally extending body portion 78. The outer surface 80 of body portion 78 is preferably conically shaped narrowing in a direction distal from the end flange 76. The seal member 72 has an inner peripheral surface 82 surrounding an interior bore for accommodating the catheter 22. With the seal member 72 in its quiescent uncompressed state, the inner surface 82 is sufficiently large to allow the member 72 to slide along the catheter outer surface 44.

The inner surface 36 of sleeve 30 tapers outwardly at 90 adjacent to the sleeve proximal end 40. The outward tapering of the surface 36 forms a wedge recess 92 for receiving the body portion 78 of the seal member 72 (Figure 4).

The sealing device 70 further includes a locking, or compression, member 100 comprising a tubular wall 102 having an outer peripheral surface 104 and an inner peripheral surface 106 surrounding a bore for accommodating the catheter 22. The compression member 100 is configured so that in its unlocked state, it can slide distally along catheter 22 into latching engagement (locked state) with the sleeve 30 while compressing the annular seal member 72 into the wedge recess 92. When so compressed, the seal member 72 seals against the catheter outer surface 44 and the sleeve inner surface 90 to prevent migration of deleterious material into the patient's body. Moreover, when the seal member 72 is compressed by the latched compression member 100, it locks the catheter to the sleeve to prevent relative movement therebetween.

For the purpose of latching to the sleeve, the compression member 100 is provided with one or more cantilevered resilient fingers 120. Each finger has a terminal projection 122 configured to snap into a groove 124 (Figure 4) formed in the sleeve outer surface 34 proximate to the sleeve proximal end 40. So, during the installation of the catheter assembly 20, the physician can slide the compression member 100 distally along catheter 22 toward the sleeve 30 to compress the seal member 72 therebetween until the projection 122 latches into groove 124. With the seal member 72 thus compressed, it acts to frictionally lock the catheter 22 to the sleeve 30.

The compression member 100 can be unlatched by pulling the resilient finger 120 radially outward withdraw to the projection 122 from the groove 124. This allows the compression member 100 to be moved proximally along the catheter thus decompressing the seal member 72 and permitting the physician to reposition the catheter or remove the catheter through the sleeve 30 to exchange it with a replacement catheter.

An anchor 130 is provided for anchoring the sleeve proximal end 40 to the patient's skin. The anchor 130 is comprised of a base 132 supporting a catheter guide 134. The guide 134 defines a bore for accommodating the catheter 22. The base is preferably provided with holes 136, 138 for suturing the anchor 130 to the patient's skin.

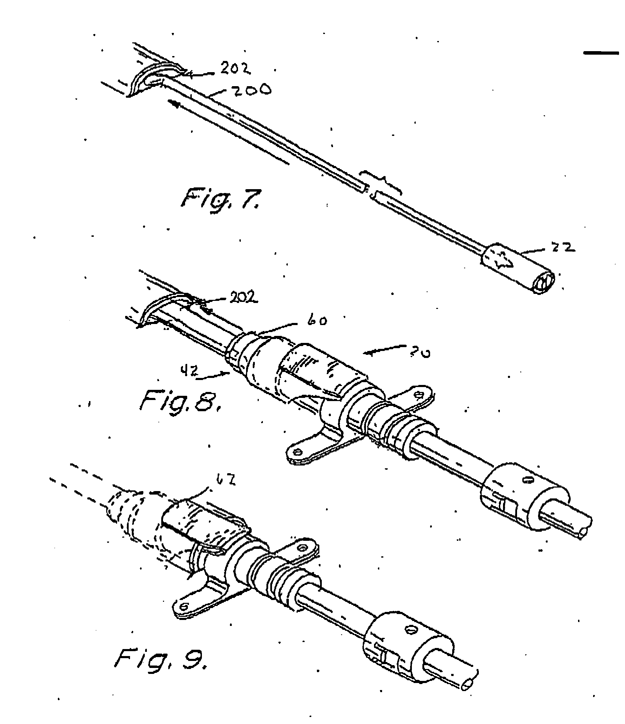

Figures 7-12 schematically depict successive steps in an exemplary procedure for implanting the catheter assembly 20 shown in Figures 1-6.

Figure 7 shows the use of a conventional tunneler tool 200 being inserted through a patient's incision 202 to form a tunnel through which the distal end of the catheter 22 is pulled by the proximal end of tool 200.

Figure 8 shows the catheter assembly 20 with the sleeve distal end 42 and protective sheath 60 being inserted through the incision 202.

Figure 9 shows the catheter assembly 20 inserted further into the incision for positioning the porous layer 50 just beneath the epidermal skin layer 52 and adjacent to the dermis layer 54. Note the protective sheath tab 62 extending outwardly from the sleeve.

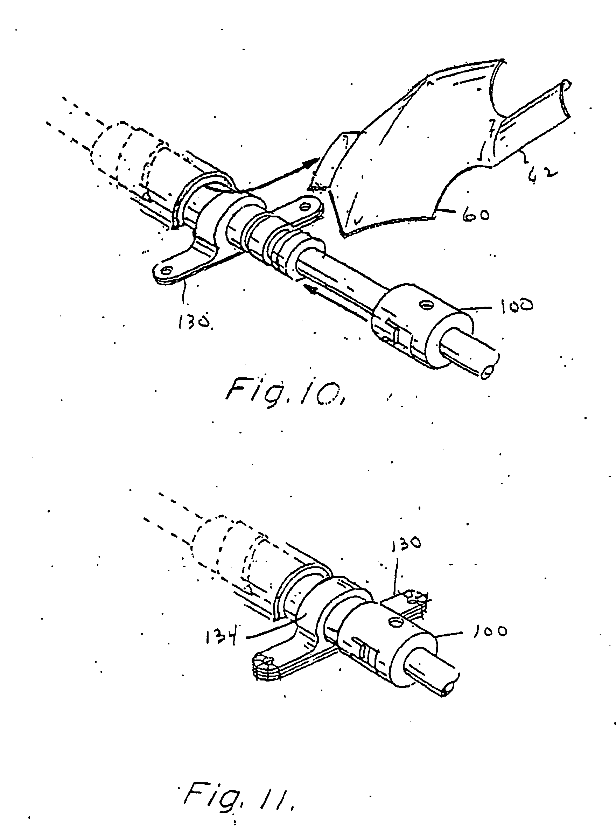

Figure 10 shows the protective sheath 62 being peeled away (as a consequence of the physician pulling tab 62) from the sleeve 30 to directly expose the porous layer 50 to the patient's dermis.

Figure 11 shows the anchor 130 sutured to the patient's skin to thus securely hold the sleeve proximal end 40 and facilitate the latching of compression member 100 to sleeve 30. Inorder to latch the compression member, the physician should first align the index marks 140 on sleeve 30 and 142 on compression member 100 (Figure3) prior to sliding member 100 against sleeve 30 to latch projection 122 into groove 124..

Figure 12 shows a cross-section of the installed catheter assembly 20 with the porous layer 50 contacting the patient's dermis 54 to promote tissue ingrowth.

After initial implantation of the catheter 22 as depicted in Figures 7-12, it is sometimes desirable to reposition the catheter for more effective treatment and/or to reduce patient discomfort. Such repositioning can involve slightly adjusting the position and/or orientation of the catheter distal end and is achieved by unlatching the compression member 100 and then sliding the catheter into or out of the sleeve and/or by rotating the catheter. In order to avoid introducing deleterious material when the catheter is pushed distally, it is desirable to create a sterile field around that portion of the catheter which can move distally past the annular seal member 72. As shown in Figure 13, an extensible seal member 200, e.g., a bellows, is provided. The seal member 200 has a distal end 202 which is sealed to the compression member 100 and a proximal end 204 which is sealed to the catheter 22.

It is further pointed out that with the compression member 100 unlatched, an implanted catheter can be fully withdrawn through the sleeve for replacement by a new catheter. Although different technique can be employed, it is contemplated that the physician will thread a guide wire through the old catheter prior to withdrawing it. The new catheter is then threaded along the guide wire and through the sleeve. When the new catheter is properly placed, the guide wire is withdrawn. The new catheter preferably carries a new seal member 72 and compression member 100.

From the foregoing, it should now be understood that a method and apparatus has been described for positioning, repositioning, and/or replacing an elongate conduit extending through a patients skin. In use, the physician will manipulate the conduit portion extending exteriorly of the patient's skin to position and orient the conduit distal end adjacent to a selected interior body site. When the distal end is properly positioned, the compression member is latched to thereby lock the conduit relative to the implanted sleeve. As previously noted, the conduit can comprise a catheter for delivering an/or extracting fluid to/from the interior body site or an electric cable for delivering and/or deriving an electric signal to/from the interior body site. In some situations, it may be desirable to use both a catheter and an electric cable. Although only a limited number of structural embodiments have been described, it is recognized that various modifications and alterations will occur to persons skilled in the art which fall within the intended scope of the invention as defined by the appended claims.

## Claims

1. An apparatus suitable for percutaneous implantation in a patient's body, said apparatus including:
an elongate sleeve (30) having first and second ends, said first end (40) residing in use above the skin, said sleeve comprising a wall (32) having a peripheral outer surface (34) and a peripheral inner surface (36) defining a passageway (38) extending from said first (40) to said second end (42); an elongate catheter (22) extending through said passageway (38);
a layer of porous material (50) mounted on said sleeve outer surface proximate to said sleeve second end, said porous material intended for placement in the dermis of a patient's body for promoting tissue ingrowth to form an infection resistant barrier;
**characterized in that** the elongate catheter (22) is configured for slidable movement with respect to the passageway (38);
the apparatus includes means for anchoring (130) said sleeve first end (40) to the outer skin surface of said patient; an annular seal member (72) mounted around said catheter (22) adjacent said sleeve first end (40); and means for sealing (100) said annular seal member against the outer surface of said catheter (22) for preventing the migration of deleterious material between said sleeve inner surface and said catheter outer surface.

2. The apparatus of claim 1 wherein said means for sealing (70) includes a compression member (100) for pressing said seal member (72) against said sleeve and said catheter (22).

3. The apparatus of claim 2 including means for selectively latching said compression member (100) for preventing catheter movement in said passageway and unlatching said compression member to permit catheter movement in said passageway.

4. The apparatus of claim 1 wherein said sleeve defines a recess (124) adjacent to said first end; and wherein
said annular seal member (72) is configured to be accommodated in said recess.

5. The apparatus of claim 2 wherein seal member (72) is formed of compressible material; and wherein
said means for sealing includes means for compressing said seal member against said sleeve (30) and said catheter (22).

6. The apparatus of claim 1 further including a protective sheath (56) carried by said sleeve (30) and covering said layer of porous material (50); and wherein
said protective sheath (56) is comprised of thin flexible material configured to be readily removed when said porous material is placed in said dermis.

7. The apparatus of claim 1 further including:
an extensible seal member (72) mounted around said catheter (22), said seal member having a distal end secured to said sleeve (30) and a proximal end secured to the outer surface of said catheter.

8. The apparatus of claim 1 wherein said layer of porous material (50) incorporates antimicrobial and/or anti-inflammatory agents.

## Patentansprüche

1. Vorrichtung, die zur perkutanen Implantation in den Körper eines Patienten geeignet ist, wobei die Vorrichtung Folgendes umfasst:
eine längliche Hülse (30) mit einem ersten und einem zweiten Ende, wobei das erste Ende (40) bei Verwendung oberhalb der Haut vorliegt, wobei die Hülse Folgendes umfasst: eine Wand (32) mit einer Umfangsaußenoberfläche (34) und einer Umfangsinnenoberfläche (36), die einen Durchlass (38) definiert, der sich von dem ersten Ende (40) zu dem zweiten Ende (42) erstreckt; einen länglichen Katheter (22), der sich durch den Durchlass (38) erstreckt;
eine Schicht poröses Material (50), die auf der Außenoberfläche der Hülse in der Nähe des zweiten Endes der Hülse aufgebracht ist, wobei das poröse Material dazu dient, in der Dermis des Körpers eines Patienten platziert zu werden, um das Hineinwachsen von Gewebe zu fördern, um eine infektionsresistente Schranke zu bilden;
**dadurch gekennzeichnet, dass** der längliche Katheter (22) für eine Gleitbewegung in Bezug auf den Durchlass (38) ausgebildet ist;
dass die Vorrichtung Mittel zur Verankerung (130) des ersten Endes (40) der Hülse an der Außenoberfläche der Haut des Patienten umfasst; dass ein ringförmiges Dichtungselement (72) um den Katheter (22) benachbart zu dem ersten Ende (40) der Hülse angebracht ist; und dass Mittel zum Abdichten (100) des ringförmigen Dichtungselements gegenüber der Außenoberfläche des Katheters (22) vorliegt, um zu verhindern, dass schädliches Material zwischen der Innenoberfläche der Hülse und der Außenoberfläche des Katheters migriert.

2. Vorrichtung nach Anspruch 1, worin das Mittel zum Abdichten (70) ein Druckelement (100) umfasst, um das Dichtungselement (72) gegen die Hülse und den Katheter (22) zu drücken.

3. Vorrichtung nach Anspruch 2, die Mittel umfasst, um das Druckelement (100), wahlweise zu verriegeln, um eine Bewegung des Katheters in dem Durchlass zu verhindern, und zu entriegeln, um eine Bewegung des Katheters in dem Durchlass zuzulassen.

4. Vorrichtung nach Anspruch 1, worin die Hülse eine zu dem ersten Ende benachbarte Ausnehmung (124) definiert und worin:
das ringförmige Dichtungselement (72) ausgebildet ist, um in dieser Ausnehmung aufgenommen zu werden.

5. Vorrichtung nach Anspruch 2, worin das Dichtungselement (72) aus einem zusammendrückbaren Material ausgebildet ist und worin:
das Mittel zum Abdichten Mittel umfasst, um das Dichtungselement gegen die Hülse (30) und den Katheter (22) zu drücken.

6. Vorrichtung nach Anspruch 1, ferner umfassend eine Schutzhülle (56), die durch die Hülse (30) getragen wird und die Schicht des porösen Materials (50) abdeckt; und worin:
die Schutzhülle (56) aus einem dünnen flexiblen Material besteht, das ausgebildet ist, um einfach entfernt werden zu können, wenn das poröse Material in der Dermis platziert wird.

7. Vorrichtung nach Anspruch 1, ferner umfassend:
ein dehnbares Dichtungselement (72), das um den Katheter (22) herum angebracht ist, wobei das Dichtungselement ein distales Ende, das an der Hülse (30) befestigt ist, und ein proximales Ende aufweist, das an der Außenoberfläche des Katheters befestigt ist.

8. Vorrichtung nach Anspruch 1, worin die Schicht des porösen Materials (50) antimikrobielle und/oder entzündungshemmende Mittel umfasst.

## Revendications

1. Appareil conçu pour l'implantation percutanée dans le corps d'un patient, ledit appareil comportant:
un manchon oblong (30) ayant des première et seconde extrémités, ladite première extrémité (40) se trouvant en cours d'utilisation au-dessus de la peau, ledit manchon comprenant une paroi (32) ayant une surface extérieure périphérique (34) et une surface périphérique intérieure (36) définissant un passage (38) s'étendant de ladite première (40) à ladite deuxième extrémité (42); un cathéter oblong (22) s'étendant à travers ledit passage (38);
une couche de matériau poreux (50) montée sur ladite surface extérieure de manchon à proximité de ladite seconde extrémité de manchon, ledit matériau poreux étant destiné à être placé dans le derme du corps d'un patient pour encourager la croissance vers l'intérieur du tissu pour former une barrière résistant à l'infection;
**caractérisé en ce que** le cathéter oblong (22) est configuré pour un mouvement coulissant par rapport au passage (38);
l'appareil comporte des moyens pour l'ancrage (130) de ladite première extrémité de manchon (40) à la surface de peau extérieure dudit patient; un élément d'étanchéité annulaire (72) monté autour dudit cathéter (22) d'une manière adjacente à ladite première extrémité de manchon (40); et un moyen pour rendre étanche (100) ledit élément d'étanchéité annulaire contre la surface extérieure dudit cathéter (22) pour empêcher la migration d'un matériau délétère entre ladite surface intérieure de manchon et ladite surface extérieure de cathéter.

2. Appareil selon la revendication 1, dans lequel ledit moyen d'étanchéité (70) comporte un élément de compression (100) pour presser ledit élément d'étanchéité (72) contre ledit manchon et ledit cathéter (22).

3. Appareil selon la revendication 2, comportant des moyens pour verrouiller sélectivement ledit élément de compression (100) afin d'empêcher un mouvement du cathéter dans ledit passage et pour déverrouiller ledit élément de compression pour permettre le mouvement du cathéter dans ledit passage.

4. Appareil selon la revendication 1, dans lequel ledit manchon définit un évidement (124) adjacent à ladite première extrémité; et où
ledit élément d'étanchéité annulaire (72) est configuré pour être logé dans ledit évidement.

5. Appareil selon la revendication 2, dans lequel l'élément d'étanchéité (72) est réalisé en matériau compressible; et où
ledit moyen d'étanchéité comporte des moyens pour compresser ledit élément d'étanchéité contre ledit manchon (30) et ledit cathéter (22).

6. Appareil selon la revendication 1, comportant en outre une gaine de protection (56) portée par ledit manchon (30) et couvrant ladite couche de matériau poreux (50); et où
ladite gaine de protection (56) est constituée d'un matériau flexible mince configuré pour être facilement retiré lorsque ledit matériau poreux est placé dans ledit derme.

7. Appareil selon la revendication 1, comportant en outre:
un élément d'étanchéité extensible (72) monté autour dudit cathéter (22), ledit élément d'étanchéité ayant une extrémité distale fixée audit manchon (30) et une extrémité proximale fixée à la surface extérieure dudit cathéter.

8. Appareil selon la revendication 1, dans lequel ladite couche de matériau poreux (50) incorpore des agents anti-microbiens et/ou anti-inflammatoires.
